# EUROPÄISCHE PATENTSCHRIFT

(11) **EP 0 699 057 B1**
(45) Veröffentlichungstag und Bekanntmachung des Hinweises auf die Patenterteilung: **31.05.2000**
(21) Anmeldenummer: 95912177.3
(22) Anmeldetag: 01.03.1995
(51) Int. Cl.: A61B 17/80, F16B 39/32

(54) **STABILISIERUNGSEINRICHTUNG, INSBESONDERE ZUR STABILISIERUNG DER WIRBELSÄULE**
STABILISING ARRANGEMENT, IN PARTICULAR FOR STABILISING THE SPINAL COLUMN
DISPOSITIF DE STABILISATION, NOTAMMENT DE LA COLONNE VERTEBRALE

(30) Priorität: 22.03.1994 DE 4409833
(43) Veröffentlichungstag der Anmeldung: 06.03.1996
(73) Patentinhaber: BIEDERMANN MOTECH GmbH, 78054 Villingen-Schwenningen (DE)
(72) Erfinder: BIEDERMANN, Lutz, D-78048 VS-Villingen (DE); HARMS, Jürgen, D-76133 Karlsruhe (DE)
(74) Vertreter: Prüfer, Lutz H., Dipl.-Phys.
(86) Internationale Anmeldenummer: EP9500745
(87) Internationale Veröffentlichungsnummer: WO9525474

(56) Entgegenhaltungen:
- EP-A- 0 242 842
- EP-A- 0 530 585
- EP-A- 0 599 640
- DE-B- 1 791 228
- DE-C- 424 324
- US-A- 3 419 057

## Beschreibung

Die Erfindung bezieht sich auf eine Stabilisierungseinrichtung, insbesondere zur Stabilisierung der Wirbelsäule.

Stabilisierungseinrichtungen werden verwendet, wenn zwei Wirbei der Wirbelsäule, insbesondere im Bereich der Halswirbelsäule, miteinander verbunden werden sollen. Herkömmliche Stabilierungseinrichtungen bestehen aus einer Stabilisierungsplatte, die mittels mindestens zweier Knochenschrauben, die jeweils in die zu verbindenden Wirbel geschraubt werden, befestigt wird.

Gerade im Bereich der Halswirbel besteht durch die Bewegung des Kopfes die Neigung, daß sich die Schrauben lockern bzw. drehen und geradezu herausgeschlagen werden. Dadurch ist eine stabile Verbindung der Wirbel nicht mehr gewährleistet.

Eine Stabilisierungseinrichtung mit den Merkmalen des Oberbegriffes des Patentanspruches 1 ist aus der DE-AS-1 791 228 bekannt.

In der Druckschrift US-A-3 419 057 wird eine Arretiereinrichtung für Gewindeschrauben beschrieben, bei der ein mit der aufzunehmenden Schraube in Eingriff gelangendes federndes Element vorhanden ist.

Es ist Aufgabe der Erfindung, eine Stabilisierungseinrichtung bereitzustellen, bei der die Schrauben gegen eine lösende Verdrehung gesichert sind.

Diese Aufgabe wird durch eine Stabilisierungseinrichtung nach Anspruch 1 gelöst.

Die Stabilisierungseinrichtung hat den Vorteil, daß die Sicherung der Schrauben gegen unbeabsichtigtes Herausdrehen so beschaffen ist, daß jede Schraube leicht in das Material hineingedreht werden kann und keine von außen anzubringende Einrichtung zum Arretieren der Knochenschraube gegen unbeabsichtigtes Herausdrehen erforderlich ist. Somit kann auf übliche Schraubensicherungstechniken, zum Beispiel mittels federnder Zahnscheiben, Abdeckklappen oder ähnlichem, die während einer Wirbeloperation eher ungünstig zu handhaben sind bzw. hoch aufbauen, verzichtet werden.

Vorteilhafte Weiterbildungen sind in den Unteransprüchen gegeben.

Es folgt die Beschreibung eines Ausführungsbeispieles anhand der Figuren.

Von den Figuren zeigen:
- Fig. 1: eine Draufsicht auf die Stabilisierungsplatte;
- Fig. 2: eine Schnittansicht entlang der Linie A - A in Fig. 1 in vergrößertem Maßstab;
- Fig. 3: eine vergrößerte Darstellung des Ausschnittes B in Fig. 1;
- Fig. 4: eine Seitenansicht einer Knochenschraube;
- Fig. 5: eine Draufsicht auf den Kopf der Knochenschraube in Fig. 4;
- Fig. 6: eine Schnittansicht, die den Zustand der Knochenschraube beim Einschrauben vor der Endbefestigung darstellt;
- Fig. 7: eine Schnittansicht, die die Knochenschraube im endgültig eingeschraubten Zustand darstellt.

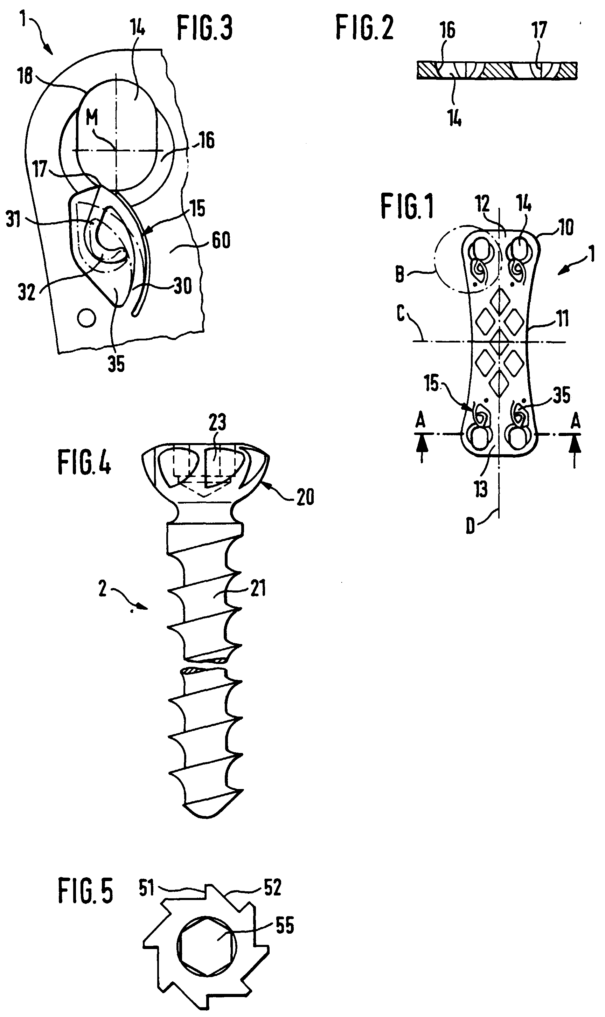

Wie aus Fig. 1 ersichtlich ist, weist die Stabilisierungseinrichtung eine im wesentlichen rechteckige Stabilisierungsplatte 1 auf. Für die weitere Beschreibung wird die in Längsrichtung des Rechtecks verlaufende Mittellinie der Platte mit D und die senkrecht dazu in Querrichtung des Rechtecks verlaufende Mittellinie mit C bezeichnet.

Die Stabilisierungsplatte 1 ist so geformt, daß ihre Ecken 10 abgerundet sind und ihre Längsseiten 11 symmetrisch zur Mittellinie D hin gekrümmt verlaufen.

Die Platte weist ein erstes Ende 12 und ein diesem gegenüberliegendes zweites Ende 13 mit jeweils zwei Bohrungen 14 zur Aufnahme von Knochenschrauben auf. Die Bohrungen 14 sind paarweise spiegelsymmetrisch zur Mittellinie C bzw. zur Mittellinie D der Platte angeordnet.

Wie in Fig. 3 gezeigt ist, ist jede Bohrung 14 als Langloch ausgebildet, dessen größerer Durchmesser parallel zur Mittellinie D ausgerichtet ist. Das Langloch ist von einem Rundloch mit einem Durchmesser, der gleich dem kleineren Durchmesser des Langlochs ist, überlagert, wobei der Mittelpunkt M des Rundloches auf der Längsachse des Langloches angeordnet ist und in Richtung zu der Mittellinie C hin gegenüber dem Mittelpunkt des Langloches verschoben ist. Das Rundloch ist mit einer Senkung 16 zur paßförmigen Aufnahme des Schraubenkopfes einer Knochenschraube 2, die später im Detail beschrieben wird, versehen.

Wie aus Fig. 1 ersichtlich ist, weist die Stabilisierungsplatte 1 an dem der Mittellinie C der Stabilisierungsplatte 1 zugewandten Ende jeder Bohrung 14 ein Arretierelement 15 zur Arretierung der Knochenschraube 2 auf.

Das Arretierelement 15 ist als ein federndes Element als Teil der Stabilisierungsplatte 1 und in der Plattenebene liegend ausgebildet. Es wird durch Herausschneiden aus der Stabilisierungsplatte gefertigt und kann in einer durch das Herausschneiden in der Stabilisierungsplatte 1 entstandenen Ausnehmung 35 in der Plattenebene ausgelenkt werden.

Wie in Fig. 3 dargestellt ist, weist das Arretierelement einen gekrümmten stegförmigen Abschnitt 30 auf, der mit seinem einen Ende in die Stabilisierungsplatte 1 übergeht. An seinem anderen Ende grenzt auf der dem Krümmungsmittelpunkt zugewandten Seite in einem spitzen Winkel ein gerader stegförmiger Abschnitt 31 an, dessen freies Ende in Richtung des gekrümmten stegförmigen Abschnitts 30 gebogen ist. Die Krümmung des gekrümmten stegförmigen Abschnittes 30 ist, betrachtet vom Ansatzpunkt der Stabilisierungsplatte aus, eine Linkskrümmung. Die durch den gekrümmten stegförmigen Abschnitt 30 und den geraden stegförmigen Abschnitt 31 gebildete Spitze 17 des Arretierelementes 15 weist einen Abstand zum Mittelpunkt M des Rundlochs auf, der kleiner als der Radius des Rundloches ist, d.h. die spitze 17 ragt etwas in das Rundloch hinein, was auch aus der Schnittansicht von Fig. 2 ersichtlich ist.

Der gerade stegförmige Abschnitt 31 verläuft radial bezüglich des Rundlochs, und schließt mit dem gekrümmten stegförmigen Abschnitt im Bereich der Spitze 17 einen Winkel von ungefähr 60° ein. Das Arretierelement 15 ist elastisch und kann in der Ausnehmung 35 der Stabilisierungsplatte 1 von der oben beschriebenen spannungsfreien Stellung (durchgezogene Linie in Fig. 3) in Richtung des Krümmungsmittelpunkts des gekrümmten stegförmigen Abschnitts 30 ausgelenkt werden (gestrichelte Linie in Fig. 3). Der gekrümmte stegförmige Abschnitt grenzt mit seiner dem Krümmungsmittelpunkt abgewandten Seite an den Plattenkörper 60 an. In der ausgelenkten, gestrichelt dargestellten Stellung weist die Spitze 17 des Arretierelements einen Abstand zum Mittelpunkt M des Rundlochs auf, der größer oder gleich dem Außenradius der das Rundloch umgebenden Senkung 16 ist.

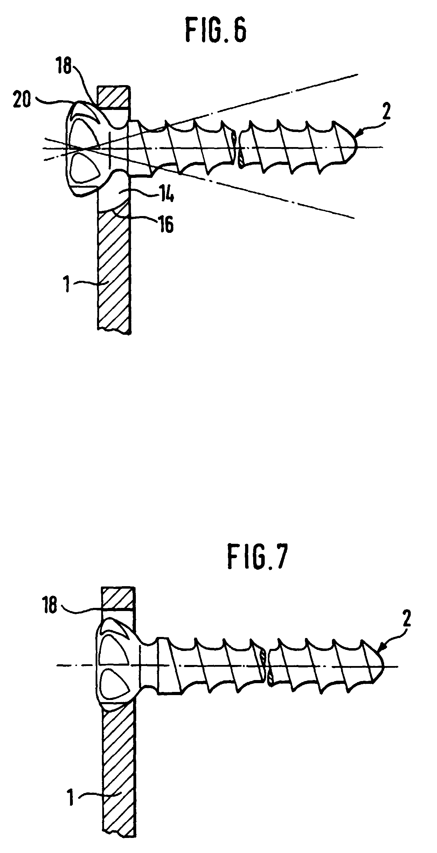

Die Stabilisierungseinrichtung weist weiter Knochenschrauben 2, wie in Fig. 4 gezeigt, zur Befestigung der Stabilisierungsplatte 1 auf. Die Knochenschraube 2 besitzt einen Schraubenkopf 20 und einen Gewindeschaft 21. Der Schraubenkopf 20 ist an einer Seite mit dem Gewindeschaft verbunden und weist auf dieser Seite einen im wesentlichen kugelabschnittförmigen Rand auf, wobei sich der Mittelpunkt des Abschnitts auf der dem Gewindeschaft abgewandten Seite und auf der Längsachse der Schraube befindet. Der Durchmesser des Schraubenkopfes ist gleich oder etwas kleiner als der Durchmesser der Senkung 16 des Rundlochs. An dem sphärisch gewölbten Rand des Schraubenkopfes 20 ist eine Verzahnung 23 vorgesehen, in die das Arretierelement 15 zur Arretierung einrasten kann.

Wie aus Fig. 5 ersichtlich ist, ist die Verzahnung 23 so ausgebildet, daß jeder Zahn definiert wird durch eine erste Zahnflanke 51, die in die radiale Richtung des Schraubenkopfes weist, und eine zweite Zahnflanke 52, die einen spitzen Winkel zu dieser Richtung bildet. Die Anordnung der zweiten Zahnflanken ist so gewählt, daß, wenn als eine erste Drehrichtung der Uhrzeigersinn betrachtet wird, die zweite Zahnflanke im Sinne dieser ersten Drehrichtung vor der ersten Zahnflanke liegt. Im eingeschraubten Zustand liegt der Schraubenkopf 20 in der Senkung 16 des Rundlochs, und der gerade stegförmige Abschnitt 31 des Arretierelements liegt an der ersten Zahnflanke 51 der Verzahnung 23 an und bildet eine Arretierung.

Auf der dem Gewindeschaft 21 abgewandten Seite ist in der Mitte des Schraubenkopfes 20 eine sechskantförmige Ausnehmung 55 zum Einführen eines Schraubenschlüssels zum Drehen der Schraube 2 vorgesehen.

Die Stabilisierungsplatte 1 und die Knochenschraube 2 sind aus einem körperfreundlichen Metall, vorzugsweise aus Titan gebildet.

Die Wirkungsweise der Arretiereinrichtung nach den Fig. 1 bis 5 ist die folgende: Beim Einschrauben in der ersten Drehrichtung im Uhrzeigersinn gleitet der Schraubenkopf 20, sobald er in den Bereich der Senkung 16 gelangt ist, mit jeder der zweiten Zahnflanken 52 an dem gekrümmten stegförmigen Abschnitt 30 des Arretierelements 15 entlang, wobei das Arretierelement durch die daran entlang gleitenden Zahnflanken 52 so ausgelenkt wird, daß der gerade stegförmige Abschnitt 31 aus seiner Arretierstellung von der ersten Zahnflanke 51 weggedrückt wird. Die Spitze 17 des Arretierelements weist dann jeweils einen Abstand zum Mittelpunkt M des Rundlochs auf, der größer als der Radius des Rundlochs ist. Nachdem jeweils die zweite Zahnflanke 52 vorbeigeglitten ist, rastet das federnd ausgebildete Arretierelement 15 in seine oben beschriebene Arretierstellung ein.

Beim Versuch des Drehens in einer zur ersten Drehrichtung entgegengesetzten zweiten Drehrichtung zum Lösen der Schraube wird die erste Zahnflanke 51 der Verzahnung gegen den geraden stegförmigen Abschnitt 31 des Arretierelements gedrückt, und die Spitze 17 des Arretierelements wird in der Verzahnung festgeklemmt. Somit ist ein Lösen der Schraube ohne zusätzliche Hilfsmittel, mit dem das Arretierelement in die gestrichelt gezeichnete Stellung ausgelenkt wird, nicht möglich.

Die Befestigung der Stabilisierungsplatte 1 soll nun anhand der Fig. 6 und 7 beschrieben werden.

Fig. 6 zeigt eine Schnittansicht eines Teiles der Stabilisierungsplatte 1 mit der Bohrung 14 und dem Zustand der Knochenschraube 2 vor der Endbefestigung. Die Schraube wird etwa im längsseitigen Mittelpunkt des Langloches aufgesetzt. Dann wird der Gewindeschaft 21 so weit eingeschraubt, bis der Schraubenkopf 20 an einer Oberkante 18 des nicht versenkten Randes des Langlochs anstößt.

Bei weiterem Drehen in der ersten Drehrichtung wird der Schraubenkopf 20 von dieser Oberkante 18 weggedrückt und in die paßförmige Senkung 16 des dein Langloch überlagerten Rundlochs hineingezogen. Dabei zentriert sich der Kopf 20 der Knochenschraube 2 in dem Rundloch, wobei die Wirbel, die jeweils mit dem ersten bzw. mit dem zweiten Ende der Stabilisierungsplatte verschraubt sind, aufeinandergepreßt werden. Fig. 7 zeigt eine Schnittansicht der Stabilisierungsplatte 1 mit der Knochenschraube 2 im vollständig eingeschraubten Zustand.

Der querseitige Durchmesser des Langloches und damit der Durchmesser des zu dem Mittelpunkt der Senkung 16 gehörenden Loches ist so relativ zum Schaft der Schraube 2 gewählt, daß die Schraube 2 in der in Fig. 6 gezeigten Weise innerhalb eines Winkels von etwa 15° um die Lochachse geschwenkt einsetzbar ist. Da die Zahnflanken sich in der in Fig. 4 bzw. Fig. 7 ersichtlichen Weise in axialer Richtung gesehen über nahezu die gesamte Höhe des Kopfes erstrecken, erfolgt die Arretierung in der oben beschriebenen Weise auch bei geneigt eingesetzter Schraube.

## Patentansprüche

1. Stabilisierungseinrichtung, insbesondere zur Stabilisierung der Wirbelsäule,
mit einer Stabilisierungsplatte (1) mit einem ersten Ende (12) und einem diesem gegenüber liegenden zweiten Ende (13)und jeweils wenigstens einer Bohrung (14) an jedem Ende zur Aufnahme von Knochenschrauben (2) , wobei die Knochenschrauben einen Gewindeschaft (21) zum Einschrauben in einer ersten Drehrichtung und Lösen in einer zweiten Drehrichtung aufweisen,
dadurch gekennzeichnet, daß eine Einrichtung zum Arretieren (15, 23) der Knochenschrauben gegen unbeabsichtigtes Drehen in der zweiten Drehrichtung vorgesehen ist, die Einrichtung (15) ein mit der aufzunehmenden Knochenschraube in Eingriff gelangendes federndes Element (30, 31, 32) aufweist und das federnde Element (30,31,32) als Teil der Stabilisierungsplatte (1) gebildet ist.

2. Stabilisierungseinrichtung nach Anspruch 1,
dadurch gekennzeichnet, daß die Knochenschraube (2) einen an den Gewindeschaft (21) angrenzenden Schraubenkopf (20) mit einer an dessen Umfang vorgesehenen Verzahnung (23) , in die das federnde Element zur Arretierung einrastet, aufweist.

3. Stabilisierungseinrichtung nach Anspruch 2,
dadurch gekennzeichnet, daß die Verzahnung (23) so ausgebildet ist, daß eine erste Zahnflanke (51) in radiale Richtung des Schraubenkopfes (20) weist und eine zweite Zahnflanke (52) einen spitzen Winkel zu dieser Richtung bildet und bezüglich der ersten Drehrichtung die zweite Zahnflanke vor der ersten Zahnflanke liegt.

4. Stabilisierungseinrichtung nach Anspruch 2 oder 3,
dadurch gekennzeichnet, daß als Bohrung (14) ein Langloch vorgesehen ist, dessen Längsachse auf die beiden Enden (12, 13) der Platte zuweist, und das von einem Rundloch gleichen Durchmessers mit einer Senkung (16) zur paßförmigen Aufnahme des Schraubenkopfes (20) überlagert ist, wobei der Mittelpunkt (M) des Rundloches auf der Längsachse des Langloches angeordnet ist und zum gegenüberliegenden Ende der Platte hin gegenüber dem Mittelpunkt des Langloches verschoben ist.

5. Stabilisierungseinrichtung nach einem der Ansprüche 1 bis 4,
dadurch gekennzeichnet, daß das federnde Element (30, 31, 32) in der Plattenebene liegend gebildet ist.

6. Stabilisierungseinrichtung nach Anspruch 5,
dadurch gekennzeichnet, daß das federnde Element einen mit der Stabilisierungsplatte (1) verbundenen, gekrümmten stegförmigen Abschnitt (30) und einen auf der Seite des Krümmungsmittelpunkts daran in einem spitzen Winkel angrenzenden, geraden stegförmigen Abschnitt (31) mit einem in Richtung des gekrümmten stegförmigen Abschnitts gebogenen freien Abschluß aufweist, wobei die Krümmung des gekrümmten stegförmigen Abschnitts (30) von dessen Ansatzpunkt an der Stabilisierungsplatte (1) aus gesehen eine Linkskrümmung ist.

7. Stabilisierungseinrichtung nach, Anspruch 5 oder 6, dadurch gekennzeichnet, daß das federnde Element in einer Ausnehmung (35) der Stabilisierungsplatte (1) in Richtung des Krümmungsmittelpunkts des gekrümmten stegförmigen Abschnitts (30) ausgelenkt werden kann.

8. Stabilisierungseinrichtung nach Anspruch 7,
dadurch gekennzeichnet, daß das federnde Element so ausgebildet ist, daß in seinem spannungsfreien Zustand eine durch den gekrümmten stegförmigen Abschnitt (30) und den geraden stegförmigen Abschnitt gebildete Spitze (17) einen Abstand vom Mittelpunkt des Rundlochs hat, der kleiner als der Radius des Rundlochs ist.

9. Stabilisierungseinrichtung nach Anspruch 7 oder 8,
dadurch gekennzeichnet, daß das federnde Element so ausgebildet ist, daß seine Spitze 17 in einer ausgelenkten Stellung einen Abstand zum Mittelpunkt des Rundloches hat, der größer oder gleich dem Außenradius der das Rundloch umgebenden Senkung (16) ist.

10. Stabilisierungseinrichtung nach einem der Ansprüche 2 bis 9,
dadurch gekennzeichnet, daß der Kopf (20) der Knochenschraube (2) einen im wesentlichen kugelabschnittförmigen Rand aufweist, wobei sich der Mittelpunkt des Kugelabschnitts auf der dem Gewindeschaft (21) abgewandten Seite befindet.

11. Stabilisierungsplatte nach Anspruch 10,
dadurch gekennzeichnet, daß die Senkung (16) des Rundlochs der Form des Schaubenkopfes (20) angepaßt ist.

12. Stabilisierungseinrichtung nach einem der Ansprüche 1 bis 11,
dadurch gekennzeichnet, daß an dem ersten (12) und dem zweiten Ende (13) der Stabilisierungsplatte (1) jeweils zwei Bohrungen (14) vorgesehen sind.

13. Stabilisierungseinrichtung nach einem der Ansprüche 1 bis 12,
dadurch gekennzeichnet, daß die Stabilisierungsplatte (1) mit der Arretiereinrichtung (15) und die Knochenschraube (2) aus körperfreundlichem Metall, vorzugsweise aus Titan, gefertigt sind.

14. Stabilisierungseinrichtung nach einem der Ansprüche 2 bis 13,
dadurch gekennzeichnet, daß die Knochenschraube (2) an ihrem dem Gewindeschaft abgewandten Ende des Schraubenkopfes (20) eine Ausnehmung (55) zum Einführen eines Schraubenschlüssels zum Drehen der Schraube aufweist.

## Claims

1. Stabilizing device, in particular for stabilizing the spinal column, with a stabilizing plate (1) with a first end (12) and a second end (13) lying opposite this and in each case at least one bore (14) on each end for accommodation of bone screws (2), the bone screws having a threaded shaft (21) for screwing in by a first direction of rotation and loosening in a second direction of rotation,
characterized in that a device for locking (15, 23) the bone screws against unintentional rotation in the second direction of rotation is provided, the device (15) has a sprung element (30, 31, 32) which engages with the bone screw to be accommodated, and the sprung element (30, 31, 32) is constructed as part of the stabilizing plate (1).

2. Stabilizing device according to claim 1,
characterized in that the bone screw (2) has a screw head (20) adjacent to the threaded shaft (21) with toothing (23) provided on the circumference thereof; in which the sprung element for locking catches.

3. Stabilizing device according to claim 2,
characterized in that the toothing (23) is constructed such that a first tooth flank (51) points in the radial direction of the screw head (20) and a second tooth flank (52) forms an acute angle with this direction and the second tooth flank lies in front of the first tooth flank in respect of the first direction of rotation.

4. Stabilizing device according to claim 2 or 3,
characterized in that an elongated hole is provided as the bore (14), the longitudinal axis of which points to the two ends (12, 13) of the plate and which is overlapped by a circular hole of the same diameter with a depression (16) for accommodation, such that it fits, of the screw head (20), the central point (M) of the circular hole being arranged on the longitudinal axis of the elongated hole and being displaced towards the opposite end of the plate with respect to the central point of the elongated hole.

5. Stabilizing device according to one of claims 1 to 4,
characterized in that the sprung element (30, 31, 32) is constructed lying in the plane of the plate.

6. Stabilizing device according to claim 5,
characterized in that the sprung element has a curved bar-like section (30) joined to the stabilizing plate (1) and, on the side of the centre of curvature, a straight bar-like section (31) adjacent thereto at an acute angle, with a free end curved in the direction of the curved bar-like section, the curvature of the curved bar-like section (30) being a left-hand curvature when viewed from the starting point thereof on the stabilizing plate (1).

7. Stabilizing device according to claim 5 or 6,
characterized in that the sprung element can be deflected in a recess (35) of the stabilizing plate (1) in the direction of the centre of curvature of the curved bar-like section (30).

8. Stabilizing device according to claim 7,
characterized in that the sprung element is constructed such that in its stress-free state a point (17) formed by the curved bar-like section (30) and the straight bar-like section is at a distance from the central point of the circular hole which is smaller than the radius of the circular hole.

9. Stabilizing device according to claim 7 or 8,
characterized in that the sprung element is constructed such that its point 17 in a deflected position is at a distance from the central point of the circular hole which is greater than or the same as the outer radius of the depression (16) surrounding the circular hole.

10. Stabilizing device according to one of claims 2 to 9,
characterized in that the head (20) of the bone screw (2) has an edge substantially in the shape of a segment of a sphere, the central point of the segment of the sphere being on the side facing away from the threaded shaft (21).

11. Stabilizing plate according to claim 10,
characterized in that the depression (16) of the circular hole is adapted to the shape of the screw head (20).

12. Stabilizing device according to one of claims 1 to 11,
characterized in that in each case two bores (14) are provided on the first (12) and the second end (13) of the stabilizing plate (1).

13. Stabilizing device according to one of claims 1 to 12,
characterized in that the stabilizing plate (1) with the locking device (15) and the bone screw (2) are produced from a body-friendly metal, preferably titanium.

14. Stabilizing device according to one of claims 2 to 13,
characterized in that the bone screw (2) has on its end of the screw head (20) facing away from the threaded shalt a recess (55) for insertion of a spanner for rotation of the screw.

## Revendications

1. Dispositif de stabilisation, en particulier de stabilisation de la colonne vertébrale, comprenant une plaque de stabilisation (1) ayant une première extrémité (12) et une seconde extrémité (13) opposée à cette dernière et respectivement au moins un alésage (14) à chaque extrémité pour le logement de vis à os (2), les vis à os présentant une tige filetée (21) pour serrer dans un premier sens de rotation et desserrer dans un second sens de rotation, caractérisé en ce qu'un dispositif d'arrêt (15, 23) des vis à os est prévu pour empêcher une rotation involontaire des vis dans le second sens de rotation, en ce que le dispositif d'arrêt (15) comprend un élément élastique (30, 31, 32) s'engageant avec la vis à os à loger et en ce que l'élément élastique (30, 31, 32) est conçu comme faisant partie de la plaque de stabilisation (1).

2. Dispositif de stabilisation selon la revendication 1, caractérisé en ce que la vis à os (2) présente une tête de vis (20) adjacente à la tige filetée (21) avec une denture (23) prévue sur le pourtour de ladite tète de vis et dans laquelle s'enclenche l'élément d'arrêt élastique.

3. Dispositif de stabilisation selon la revendication 2, caractérisé en ce que la denture (23) est conçue de telle sorte qu'un premier flanc de dent (51) est orienté dans le sens radial de la tête de vis (20) et qu'un second flanc de dent (52) forme un angle aigu par rapport à ce sens et en ce que par rapport au premier sens de rotation, le second flanc de dent se trouve avant le premier flanc de dent.

4. Dispositif de stabilisation selon la revendication 2 ou 3, caractérisé en ce que l'alésage (14) est un trou oblong dont l'axe longitudinal s'étend jusqu'aux deux extrémités (12, 13) de la plaque et en ce qu'à ce trou oblong se superpose un trou rond de même diamètre avec un évidement (16) pour le logement ajusté de la tête de vis (20), le centre (M) du trou rond étant disposé sur l'axe longitudinal du trou oblong et étant déplacé vers l'extrémité opposée de la plaque par rapport au centre du trou oblong.

5. Dispositif de stabilisation selon l'une quelconque des revendications 1 à 4, caractérisé en ce que l'élément élastique (30, 31, 32) est formé dans le plan de la plaque.

6. Dispositif de stabilisation selon la revendication 5, caractérisé en ce que l'élément élastique présente un segment courbe (30), en forme de nervure, relié à la plaque de stabilisation (1) et un segment (31) droit, en forme de nervure et adjacent à ce dernier du côté du centre de courbure, en formant un angle aigu avec une extrémité libre coudée en direction du segment courbe, la courbure du segment courbe (30) étant une courbure à gauche, considérée à partir du point de départ du segment (30) sur la plaque de stabilisation (1).

7. Dispositif de stabilisation selon la revendication 5 ou 6, caractérisé en ce que l'élément élastique peut être écarté dans une cavité (35) de la plaque de stabilisation (1) en direction du centre de courbure du segment courbe (30).

8. Dispositif de stabilisation selon la revendication 7, caractérisé en ce que l'élément élastique est conçu de telle sorte que, lorsqu'il est à l'état détendu, une pointe (17) formée par le segment courbe (30) et le segment droit (31) se trouve a une distance par rapport au centre du trou rond qui est plus petite que le rayon du trou rond.

9. Dispositif de stabilisation selon la revendication 7 ou 8, caractérisé en ce que l'élément élastique est conçu de telle sorte que, dans sa position écartée, la pointe (17) se trouve à une distance par rapport au centre du trou rond, qui est plus grande ou égale au rayon extérieur de l'évidement (16) entourant le trou rond.

10. Dispositif de stabilisation selon l'une quelconque des revendications 1 à 9, caractérisé en ce que la tête (20) de la vis à os (2) présente un bord sensiblement en forme de portion sphérique dont le centre se trouve sur le côté opposé à la tige filetée (21).

11. Dispositif de stabilisation selon la revendication 10, caractérisé en ce que l'évidement (16) du trou rond est ajusté à la forme de la tête de vis (20).

12. Dispositif de stabilisation selon l'une quelconque des revendications 1 à 11, caractérisé en ce que deux alésages (14) sont prévus à la première extrémité (12) et deux alésages à la seconde extrémité (13) de la plaque de stabilisation (1).

13. Dispositif de stabilisation selon l'une quelconque des revendications 1 à 12, caractérisé en ce que la plaque de stabilisation (1) avec l'élément d'arrêt (15) et la vis à os (2) sont fabriquées en métal compatible avec le corps, de préférence en titane.

14. Dispositif de stabilisation selon l'une quelconque des revendications 2 à 13, caractérisé en ce que la vis à os (2) présente à l'extrémité de la tête de vis (20) opposée à la tige filetée, un creux (55) pour l'introduction d'une clé de serrage permettant de tourner la vis.
